# EUROPÄISCHE PATENTSCHRIFT

(11) **EP 1 772 680 B1**
(45) Veröffentlichungstag und Bekanntmachung des Hinweises auf die Patenterteilung: **05.03.2014**
(21) Anmeldenummer: 06019076.6
(22) Anmeldetag: 12.09.2006
(51) Int. Cl.: F24F 3/16

(54) **Vorrichtung und Verfahren zur Aufbereitung eines in einem Luftführungskanal geführten Luftstroms einer Klimaanlage**
Device and method for controlling an air flow in an air channel of an air conditioning unit
Dispositif et procédé de traitement d'air dans un canal de conduite d'un dispositif de climatisation

(30) Priorität: 07.10.2005 DE 102005048229
(43) Veröffentlichungstag der Anmeldung: 11.04.2007
(73) Patentinhaber: Behr GmbH & Co. KG, 70469 Stuttgart (DE)
(72) Erfinder: Boschert, Björn, Dipl.-Ing., 71254 Ditzingen (DE); Heberle, Arthur, Dr.-Ing., 68163 Mannheim (DE)
(74) Vertreter: Grauel, Andreas

(56) Entgegenhaltungen:
- EP-A- 1 079 183
- DE-A- 3 637 702
- JP-A- 2 302 536
- US-A- 3 750 556
- US-A- 5 904 896
- US-A1- 2005 168 907

## Beschreibung

Die Erfindung betrifft eine Klimaanlage mit einer Vorrichtung zur Aufbereitung eines in einem Luftführungskanal geführten Luftstroms, insbesondere zur Reduktion von Schadstoffen, Gerüchen und Keimen in dem Luftstrom. Darüber hinaus betrifft die Erfindung ein Verfahren zur Aufbereitung des Luftstroms einer Klimaanlage. Bekannte Verfahren und Vorrichtungen sind, beispielsweise, in DE-A-3 637 702 beschrieben.

Aus der DE 199 19 623 A1 ist ein Luftaufbereitungssystem zum wirksamen Abbau von Gerüchen, Keimen und Schadstoffen bekannt. Dabei wird gasförmiges Ozon als starkes Oxidationsmittel zur Abtötung von Keimen und Oxidieren von Geruchsstoffen verwendet. Dazu umfasst das Luftaufbereitungssystem eine lonisierungskammer und einen Ozonkatalysator. Ein solches Luftaufbereitungssystem wird in einer Klimaanlage zur Entkeimung des Verdampfers eingesetzt. Dazu wird das Luftaufbereitungssystem an einer geeigneten Stelle in die Klimaanlage integriert und vom Luftstrom umströmt. Die Steuerelektronik ist außerhalb der Klimaanlage angeordnet.

Nachteilig dabei ist, dass die Zuführung des Ozons sehr punktförmig geschieht. Bedingt durch die hohen Strömungsgeschwindigkeiten im Luftführungskanal kann das Ozon nicht ausreichend über den Kanalquerschnitt verteilt werden. Dies führt zu einer nicht ausreichenden Entkeimung des Verdampfers.

Darüber hinaus kommt es bedingt durch die Integration des Luftaufbereitungssystems in dem Luftführungskanal zu einer Verschmutzung des Luftaufbereitungsmoduls. Zudem führt die integrierte Ausführung des Luftaufbereitungssystems zu einem erhöhten Druckabfall der Hauptluftströmung. Ferner kann es zu einer Verschlechterung der Geräuschentwicklung kommen.

Der Erfindung liegt daher die Aufgabe zugrunde, eine Vorrichtung und ein Verfahren zur Aufbereitung eines Luftstroms einer Klimaanlage anzugeben, welches eine Verbesserung der Luftaufbereitung gegenüber dem Stand der Technik ermöglicht.

Hinsichtlich der Vorrichtung wird die Aufgabe erfindungsgemäß gelöst durch die Merkmale des Anspruchs 1. Die Aufgabe hinsichtlich des Verfahrens wird erfindungsgemäß gelöst durch die Merkmale des Anspruchs.

Vorteilhafte Weiterbildungen der Erfindung sind Gegenstand der Unteransprüche.

Dadurch, dass ein Luftaufbereitungsmodul nicht integraler Bestandteil der Klimaanlage ist, sondern in einer Abzweigleitung vom Luftführungskanal für den Hauptluftstrom angeordnet ist, sind keine Komponenten in der Hauptströmung angeordnet. Dies führt zu einer Reduzierung des Druckabfalls in der Hauptströmung gegenüber der aus dem Stand der Technik bekannten integrierten Ausführung des Luftaufbereitungsmoduls. Darüber hinaus wird der mit einer gasförmigen Substanz beaufschlagte Teilluftstrom über den gesamten Querschnitt des Luftführungskanals in den Hauptluftstrom wiedereingeführt und homogen verteilt. Hierdurch ist eine konstante und hinreichend gute Beseitigung von luftgetragenen Keimen und eine hinreichend gute Entfernung von Gerüchen und gasförmigen chemischen Verbindungen aus dem Luftstrom durch Oxidation ermöglicht. Ein weiterer Vorteil besteht darin, dass das Luftaufbereitungsmodul in Verbindung mit herkömmlichen Klimaanlagen und Belüftungsanlagen eingesetzt werden kann. Dabei eignet sich das Luftaufbereitungsmodul zum Nachrüsten in eine bestehende Anlage.

Zweckmäßigerweise ist das Luftaufbereitungsmodul als ein Ozonmodul und/oder ein Ionisationsmodul ausgebildet. Das Ozonmodul erzeugt dabei in herkömmlicher Art und Weise als starkes Oxidationsmittel Ozon, das der Entkeimung dient, indem luftgetragene Schadstoffe oxidiert und Keime abgetötet werden. Hierzu wird dem Teilluftstrom als gasförmige Substanz Ozon zugeführt. Mittels eines zusätzlichen lonisationsmoduls kann der abgezweigte Teilluftstrom darüber hinaus zum Abbau von Keimen und Schadstoffen ionisiert werden. Je nach Ausführung des Luftaufbereitungsmoduls kann dieses zusätzlich einen Aktiv-Kohle-Filter umfassen, der dem Ozonmodul nachgeschaltet ist. Hierdurch wird das Ozon gebunden und der mit dem Ozon versetzte Teilluftstrom nachgereinigt.

Für eine wirksame Entkeimung des Verdampfers ist der gasdurchsetzte Teilluftstrom in den Hauptluftstrom vor einem Verdampfer der Klimaanlage einführbar. Mit anderen Worten: Die in Art eines Bypasses ausgeführte Abzweigleitung mit dem darin angeordneten Luftaufbereitungsmodul ist vor dem Verdampfer parallel zum Luftführungskanal der Klimaanlage angeordnet. Vorzugsweise wird die Abzweigleitung unmittelbar nach dem Gebläse der Klimaanlage abgezweigt.

Für eine homogene Verteilung des gasdurchsetzten Teilluftstroms ist dieser über mehrere Strömungseingänge dem Hauptluftstrom zuführbar. In einer möglichen Ausführungsform ist hierzu der Luftführungskanal von einer Ringleitung mit mehreren in den Luftführungskanal mündenden Strömungseingängen umgeben. Zur Verteilung des gasdurchsetzten Teilluftstroms über die gesamte Ringleitung ist ein Gebläse vorgesehen. Dabei wird der gasdurchsetzte Teilluftstrom über die über den gesamten Umfang der Ringleitung, insbesondere symmetrisch, verteilten Strömungseingänge, die beispielsweise düsenförmig ausgebildet sind, unter Druck in den Hauptluftstrom wiedereingeführt. Auch kann die Ringleitung durch eine beliebig andere gestaltete Form von Kanälen oder Luftführungen ersetzt werden.

Anstelle eines Hilfsgebläses kann in einer weiteren Ausführungsform der Teilluftstrom vor einem einen Druckabfall bewirkenden Bauteil der Klimaanlage in die Abzweigleitung abzweigt und nach der Anreichung der Luft mit der gasförmigen Substanz, insbesondere mit Ozon, dem Hauptluftstrom wiederzugeführt werden. Durch das Druckgefälle stellt sich hierbei eine Strömung ein. Auch ist es möglich zur Durchströmung der Abzweig- oder Bypassleitung den dynamischen Druckanteil der Hauptluftströmung zu nutzen, um ein Teilluftstrom an einer geeigneten Stelle des Luftführungskanals abzuzweigen.

Die mit der Erfindung erzielten Vorteile bestehen insbesondere darin, dass durch die Anordnung des Luftaufbereitungsmoduls außerhalb der Klimaanlage in einer Abzweig- oder Bypassleitung das Luftaufbereitungsmodul nicht durch Umströmung von Luft verschmutzt wird. Zudem kann die Zuführung des beispielsweise mit Ozon aufbereiteten Teilluftstromes derart gestaltet werden, dass der mit Ozon versetzte Teilluftstrom homogen in der Hauptluftströmung verteilt wird, so dass beispielsweise der Verdampfer gleichmäßig und über die gesamte Oberfläche mit der aufbereitete Luft beaufschlagt wird. Hierdurch ist eine verbesserte Entkeimung des Verdampfers gegeben.

Ausführungsbeispiele der Erfindung werden anhand einer Zeichnung näher erläutert. Darin zeigen:
- Fig. 1: schematisch eine mögliche Ausführungsform für ein in einer Abzweigleitung angeordnetes Luftaufbereitungsmodul für eine Klimaanlage,
- Fig. 2: schematisch die um den Luftführungskanal der Klimaanlage angeordnete Abzweigleitung im Querschnitt, und
- Fig. 3, 4: schematisch alternative Anordnungen für die Abzweigleitung des Luftaufbereitungsmoduls.

Einander entsprechende Teile sind in allen Figuren mit den gleichen Bezugszeichen versehen.

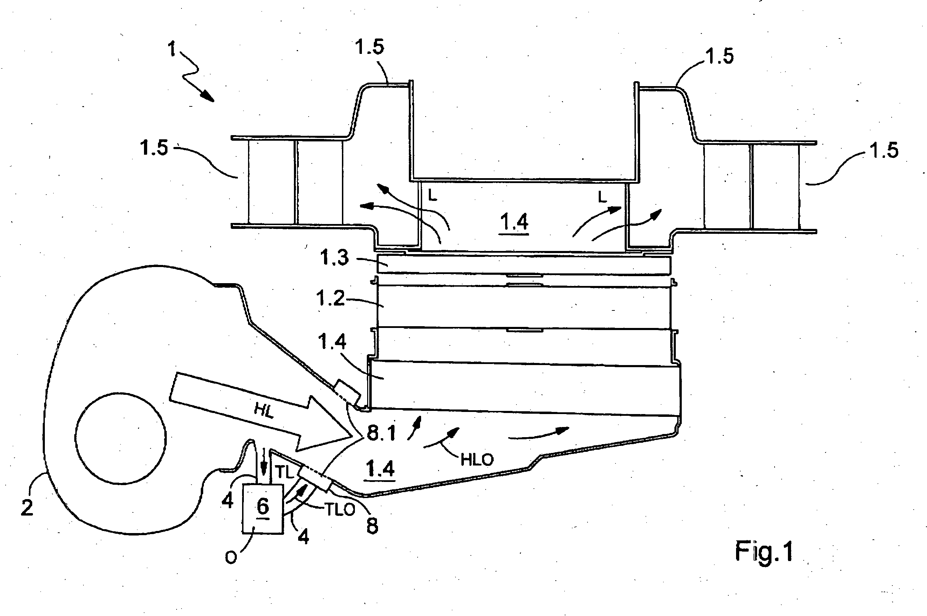

Figur 1 zeigt in schematischer Darstellung eine Klimaanlage 1 mit mehreren Klimaanlagenmodulen 1.1 bis 1.4, z. B. einem Verdampfer 1.1, einer Heizeinrichtung 1.2, gegebenenfalls einem Filter 1.3 und einem Luftführungskanal 1.4. Die Klimaanlage 1 erstreckt sich beispielsweise in wenigstens zwei Ausrichtungen.

In Figur 1 ist ein Ausführungsbeispiel für eine Erstreckung der Klimaanlage 1 in X- und in Y-Richtung. Unter X-Richtung wird dabei die Ausrichtung der Klimaanlage entlang der Fahrzeuglängsachse verstanden. Unter Y-Richtung wird die Ausrichtung entlang der Fahrzeugquerachse verstanden. Unter Z-Richtung wird die Ausrichtung der Klimaanlage 1 entlang der Vertikalachse nach oben oder nach unten verstanden.

Bevorzugt erstreckt sich die Klimaanlage 1, insbesondere deren Luftführungskanal 1.4 strömungsausgangsseitig in Querrichtung des Fahrzeugs, d.h. in Y-Richtung. Der Luftführungskanal 1.4 ist hierzu beispielsweise als Querträger zwischen den A-Säulen des Fahrzeugs angeordnet. Über Strömungsauslässe 1.5 wird die im Verdampfer 1.1 konditionierte und gegebenenfalls mittels der Heizeinrichtung 1,2 erwärmte Luft L dem Fahrzeuginnenraum im Frontbereich, Fondbereich, Heckbereich, Fußbereich und/oder im Seitenbereich zugeführt. Strömungseingangsseitig oder saugseitig verläuft der Luftführungskanal 1.4 von einem Gebläse 2 kommend in X-Richtung in den Verdampfer 1.1.

Vom Gebläse 2 angesaugte Frischluft oder Umluft wird dabei als Hauptluftstrom HL strömungseingangsseitig in den Luftführungskanal 1.4 geführt. Zum wirksamen Abbau von Gerüchen, Keimen und Schadstoffen in der dem Fahrzeuginnenraum zuzuführender Luft L wird dem Hauptluftstrom HL strömungseingangsseitig im Luftführungskanal 1.4 vor dem Verdampfer 1,1 eine gasförmige Substanz, insbesondere Ozon O zugeführt. Hierzu wird ein Teilluftstrom TL vom Hauptluftstrom HL über eine Abzweigleitung 4 abgezweigt und einem Luftaufbereitungsmodul 6 zugeführt. Die Abzweigleitung 4 ist in Art einer Bypass-Leitung ausgeführt.

Das Luftaufbereitungsmodul 6 ist beispielsweise als ein herkömmliches O-zonmodul zur Erzeugung von Ozon O ausgeführt, dass dem abgezweigten Teilluftstrom TL das Ozon O zugibt. Ein herkömmliches Ozonmodul ist beispielsweise aus der DE 199 19 623 A1 bekannt. Der mit dem Ozon O versetzte Teilluftstrom TLO wird dem Luftführungskanal 1.4 und somit dem Hauptluftstrom HL vor dem Verdampfer 1.1 wieder zugeführt wird. Zusätzlich kann das Luftaufbereitungsmodul 6 in nicht näher dargestellter Art und Weise ein lonisationsmodul zur lonisierung der Luft und/oder eine Filtereinheit zum Filtern der Luft umfassen.

Für eine homogene Verteilung des mit dem Ozon O versetzten Teilluftstroms TLO im Hauptluftstrom HL mündet die Abzweigleitung 4 in mindestens einen Strömungseingang 8.1 für den Luftführungskanal 4. Der Strömungseingang 8.1 ist beispielsweise als eine mit mehreren Öffnungen 8.1 versehene Ringleitung 8 ausgeführt, welche den Luftführungskanal 1.4 umgibt. Die Ringleitung 8 ist an einer vorgegebenen und die Strömungsverhältnisse des Hauptluftstroms HL berücksichtigenden Stelle an dem Luftführungskanal 4 angeordnet, so dass sich der Hauptluftstrom HL und der ozonversetzte Teilluftstrom TLO hinreichend gut zu einem ozonversetzten Hauptluftstrom HLO vermischen und die strömungsausgangsseitig nachfolgenden Bauteile oder Komponenten der Klimaanlage 1, insbesondere der Verdampfer 1.1 weitgehend über die gesamte Oberfläche mit dem Gemisch aus Hauptluftstrom HL und ozonversetzten Teilluftstrom TLO beaufschlagt werden. Hierdurch ist eine konstante Entkeimung des Verdampfers 1.1 sichergestellt.

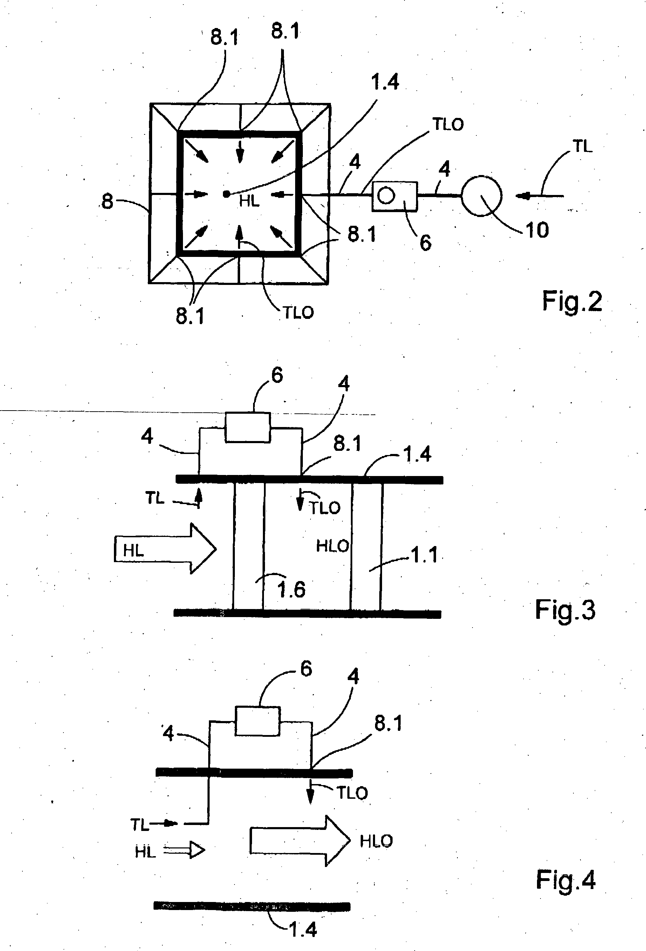

Figur 2 zeigt die um den Luftführungskanal 1.4 angeordnete Ringleitung 8 im Querschnitt. Für eine gleichmäßige Verteilung des mit dem Ozon O versetzten Teilluftstroms TLO im Hauptluftstrom HL sind über den gesamten Umfang der Ringleitung 8, insbesondere gleichmäßig verteilt, mehrere Öffnungen 8.1 in Art von Eindüsstellen vorgesehen, über die der ozonversetzte Teilluftstrom TLO dem Hauptluftstrom HL zugeführt wird. Zur Verteilung des ozonversetzten Teilluftstroms TLO kann zusätzlich in die Abzweigleitung 4 ein weiteres Hilfsgebläse 10 vor oder nach dem Luftaufbereitungsmodul 6 angeordnet sein. Alternativ kann die Ringleitung 8 durch geeignete Kanäle oder anderweitige Luftführungen ersetzt werden.

Anstelle des Hilfsgebläses 10 kann der Teilluftstrom TL an einer geeigneten Stelle des Luftführungskanals 1.4, insbesondere vor einem Druckabfall behaftetem Bauteil der Klimaanlage 1, z. B. einem Luftfilter 1.6, abgezweigt werden. Die Zuführung des ozonversetzten Teilluftstroms TLO erfolgt dabei nach dem Luftfilter 1.6 und vor dem Verdampfer 1.1. Dieses Ausführungsbeispiel ist in der Figur 3 schematisch dargestellt. Alternativ kann das Luftaufbereitungsmodul 6 in nicht näher dargestellter Art und Weise zwischen dem Luftfilter 1.6 und dem Verdampfer 1.1 in einer Abzweigleitung 4 angeordnet sein.

Ein weiteres Ausführungsbeispiel ist in der Figur 4 dargestellt. Dabei zweigt die Abzweigleitung 4 in einem Bereich des Luftführungskanals 1.4 ab, der ein entsprechendes Druckgefälle aufweist, so dass der Teilluftstrom TL aus dem Hauptluftstrom HL abgezweigt werden kann. Auch in diesem Ausführungsbeispiel kann auf ein Hilfsgebläse verzichtet werden. Die Zuführung des ozonversetzten Teilluftstroms TL erfolgt vorzugsweise vor dem Verdampfer 1.1 über ein oder mehrere Strömungseingänge 8.1.

## Patentansprüche

1. Klimaanlage mit einer Vorrichtung zur Aufbereitung eines in einem Luftführungskanal (1.4) geführten Luftstroms (HL), utmfassend mindestens ein Luftaufbereitungsmodul (6), das in einer Abzweigleitung (4) der Klimaanlage (1) angeordnet ist, wobei ein Teilluftstrom (TL) in die Abzweigleitung (4) führbar und in dem Luftaufbereitungsmödul (6) mit einer gasförmigen Substanz (O) beaufschlagbar ist, wobei der mit der gasförmigen Substanz (O) versetzte Teilluftstrom (TLO) über einen Strömungseingang (8) in den Luftführungskanal (1.4) und den Hauptluftstrom (HL) einführbar ist und sich der gasdurchsetzte Teilluftstrom (TLO) mit dem Hauptluftstrom (HL) in dem Luftführungskanal (1.4) derart vermischt, dass ein Bauteil (1.1) der Klimaanlage (1) mit dem gasdurchsetzten Hauptluftstrom (HLO) homogen beaufschlagbar ist, **dadurch gekennzeichnet, dass** der gasdurchsetzte Teilluftstrom (TLO) über mehrere Strömungseingänge (8.1) dem Hauptluftstrom (HL) zuführbar ist.

2. Klimaanlage nach Anspruch 1, wobei der gasdurchsetzte Teilluftstrom (TLO) in den Hauptluftstrom (HL) vor einem als Verdampfer (1.1) ausgebildeten Bauteil der Klimaanlage (1) einführbar ist.

3. Klimaanlage nach einem der Ansprüche 1 bis 2, wobei der Luftführungskanal (1.4) von einer Ringleitung (8) mit mehreren in den Luftführungskanal (1.4) mündenden Strömungseingängen (8.1) umgeben ist.

4. Klimaanlage nach Anspruch 3, wobei der gasdurchsetzte Teilluftstrom (TLO) mittels eines Hilfsgebläses (10) über die Ringleitung (8) verteilbär ist.

5. Klimaanlage nach einem der Ansprüche 1 bis 4, wobei die Abzweigleitung (4) vor einem einen Druckabfall bewirkenden Bauteil (1.6) der Klimaanlage (1) abzweigt.

6. Klimaanlage nach einem der Ansprüche 1 bis 5, wobei die Abzweigleitung (4) von einem Bereich des Luftführungskanals (1.4) abzweigt, der einen dynamischen Druckabfall aufweist.

7. Klimaanlage nach einem der Ansprüche 1 bis 6, wobei das Luftaufbereitungsmodul (6) als ein Ozonmodul und/oder ein Ionisierungsmodul ausgebildet ist.

8. Verfahren zur Aufbereitung eines in einem Luftführungskanal (1.4) geführten Luftstroms einer Klimaanlage (1), bei dem ein Teilluftstrom (TL) eines Hauptluftstroms (HL) in eine Abzweigleitung (4) abgezweigt und in einem in der Abzweigleitung (4) angeordneten Luftaüfbereitungsmodul (6) mit einer gasförmigen Substanz (O) beaufschlagt wird, wobei der mit der gasförmigen Substanz (O) durchsetzte Teilluftstrom (TLO) über mindestens einen Striomungseingang (8.1) in den Hauptluftstrom (HL) der Klimaanlage (1) wieder eingeführt wird und sich der gasdurchsetzte Teilluftstrom (TLO) mit dem Hauptluftstrom (HL) in dem Luftführungskanal (1.4) derart vermischt, dass ein Bauteil (1.1) der Klimaanlage (1) mit dem gasdurchsetzten Hauptluftstrom (HLO) homogen beaufschlagt wird, **dadurch gekennzeichnet, dass** der gasdurchsetzte Teilluftstrom (TLO) über mehrere Strömungseingänge (8.1) dem Hauptluftstrom (HL) zugeführt wird.

9. Verfahren nach Anspruch 8, wobei der gasdurchsetzte Teilluftstrom (TLO) in den Hauptluftstrom (HL) vor einem als Verdampfer (1.1) ausgebildeten Bauteil der Klimaanlage (1) eingeführt wird.

10. Verfahren nach einem der Ansprüche 8 bis 9, wobei der gasdurchsetzte Teilluftstrom (TLO) mittels eines Hilfsgebläses (10) über eine Ringleitung (8) verteilt und über mehrere in der Ringleitung (8) angeordnete Düsen (8.1) in den Luftführungskanal (1.4) eingeführt wird.

11. Verfahren nach einem der Ansprüche 8 bis 10, wobei der Teilluftstrom (TL) in die Abzweigleitung (4) vor einem einen Druckabfall bewirkenden Bauteil (1.6) der Klimaanlage (1) abgezweigt wird.

12. Verfahren nach einem der Ansprüche 8 bis 11, wobei der Teilluftstrom (TL) in die Abzweigleitung (4) von einem Bereich des Luftführungskanals (1.4) abgezweigt wird, der einen dynamischen Druckabfall aufweist.

## Claims

1. An air conditioning unit having a device for treating an air flow (HL) that is conducted in an air guide channel (1.4), comprising at least one air treatment module (6), which is arranged in a branch line (4) of the air conditioning unit (1), a partial air flow (TL) being conductable in the branch line (4) and exposable to a gaseous substance (0) in the air treatment module (6), the partial air flow (TLO) that is mixed with the gaseous substance (0) being introducible via a flow inlet (8) into the air guide channel (1.4) and the main air flow (HL), and the gas-permeated partial air flow (TLO) being mixed with the main air flow (HL) in the air guide channel (1.4) so that a component (1.1) of the air conditioning unit (1) can be homogeneously exposed to the gas-permeated main air flow (HLO), **characterized in that** the gas-permeated partial air flow (TLO) can be supplied to the main air flow (HL) via multiple flow inlets (8.1).

2. The air conditioning unit according to claim 1, wherein the gas-permeated partial air flow (TLO) can be introduced into the main air flow (HL) upstream of a component of the air conditioning unit (1) which is designed as an evaporator (1.1).

3. The air conditioning unit according to either claim 1 or 2, wherein the air guide channel (1.4) is surrounded by a closed loop (8) having multiple flow inlets (8.1) opening into the air guide channel (1.4).

4. The air conditioning unit according to claim 3, wherein the gas-permeated partial air flow (TLO) can be distributed over the closed loop (8) by way of an auxiliary fan (10).

5. An air conditioning unit according to any one of claims 1 to 4, wherein the branch line (4) branches off upstream of a component (1.6) of the air conditioning unit (1) which causes a pressure drop.

6. An air conditioning unit according to any one of claims 1 to 5, wherein the branch line (4) branches off a region of the air guide channel (1.4) which has a dynamic pressure drop.

7. An air conditioning unit according to any one of claims 1 to 6, wherein the air treatment module (6) is designed as an ozone module and/or an ionization module.

8. A method for treating an air flow of an air conditioning unit (1) which is conducted in an air guide channel (1.4), in which a partial air flow (TL) of a main air flow (HL) is branched off into a branch line (4) and exposed to a gaseous substance (0) in an air treatment module (6) arranged in the branch line (4), the partial air flow (TLO) that is permeated with the gaseous substance (O) being re-introduced into the main air flow (HL) of the air conditioning unit (1) via at least one flow inlet (8.1), and the gas-permeated partial air flow (TLO) being mixed with the main air flow (HL) in the air guide channel (1.4) so that a component (1.1) of the air conditioning unit (1) is homogeneously exposed to the gas-permeated main air flow (HLO), **characterized in that** the gas-permeated partial air flow (TLO) is supplied to the main air flow (HL) via multiple flow inlets (8.1).

9. The method according to claim 8, wherein the gas-permeated partial air flow (TLO) is introduced into the main air flow (HL) upstream of a component of the air conditioning unit (1) which is designed as an evaporator (1.1).

10. The method according to either claim 8 or 9, wherein the gas-permeated partial air flow (TLO) is distributed over a closed loop (8) by way of an auxiliary fan (10) and is introduced into the air guide channel (1.4) via multiple nozzles (8.1) arranged in the closed loop (8).

11. A method according to any one of claims 8 to 10, wherein the partial air flow (TL) is branched off into the branch line (4) upstream of a component (1.6) of the air conditioning unit (1) which causes a pressure drop.

12. A method according to any one of claims 8 to 11, wherein the partial air flow (TL) is branched off into the branch line (4) from a region of the air guide channel (1.4) which has a dynamic pressure drop.

## Revendications

1. Système de climatisation comprenant un dispositif servant au traitement d'un flux d'air (HL) guidé dans un conduit de guidage d'air (1.4), comprenant au moins un module de traitement d'air (6) qui est disposé dans une conduite de dérivation (4) du système de climatisation (1), où un flux d'air partiel (TL) peut être guidé dans la conduite de dérivation (4) et peut être fourni, avec une substance gazeuse (O), dans le module de traitement d'air (6), où le flux d'air partiel (TLO) mélangé avec la substance gazeuse (0) peut être introduit, par une entrée d'écoulement (8), dans le conduit de guidage d'air (1.4) et dans le flux d'air principal (HL), et le flux d'air partiel (TLO) chargé en gaz est mélangé avec le flux d'air principal (HL), dans le conduit de guidage d'air (1.4), de manière telle qu'un composant (1.1) du système de climatisation (1) puisse être alimenté, de façon homogène, avec le flux d'air principal (HLO) chargé en gaz, **caractérisé en ce que** le flux d'air partiel (TLO) chargé en gaz peut être fourni au flux d'air principal (HL) par plusieurs entrées d'écoulement (8.1).

2. Système de climatisation selon la revendication 1, où le flux d'air partiel (TLO) chargé en gaz peut être introduit dans le flux d'air principal (HL), en amont d'un composant du système de climatisation (1), configuré comme un évaporateur (1.1).

3. Système de climatisation selon l'une ou l'autre des revendications 1 et 2, où le conduit de guidage d'air (1.4) est entouré par une conduite annulaire (8) comprenant plusieurs entrées d'écoulement (8.1) débouchant dans le conduit de guidage d'air (1.4).

4. Système de climatisation selon la revendication 3, où le flux d'air partiel (TLO) chargé en gaz peut être réparti sur la conduite annulaire (8), au moyen d'un ventilateur auxiliaire (10).

5. Système de climatisation selon l'une quelconque des revendications 1 à 4, où la conduite de dérivation (4) bifurque en amont d'un composant (1.6) du système de climatisation (1), provoquant une chute de pression.

6. Système de climatisation selon l'une quelconque des revendications 1 à 5, où la conduite de dérivation (4) bifurque à partir d'une zone du conduit de guidage d'air (1.4) qui présente une chute de pression dynamique.

7. Système de climatisation selon l'une quelconque des revendications 1 à 6, où le module de traitement d'air (6) est configuré comme un module d'ozone et/ou comme un module d'ionisation.

8. Procédé de traitement d'un flux d'air d'un système de climatisation (1), guidé dans un conduit de guidage d'air (1.4), procédé dans lequel un flux d'air partiel (TL) d'un flux d'air principal (HL) est dévié dans une conduite de dérivation (4) et est fourni, avec une substance gazeuse (0), dans un module de traitement d'air (6) disposé dans la conduite de dérivation (4), où le flux d'air partiel (TLO) chargé avec la substance gazeuse (0) est introduit à nouveau, par au moins une entrée d'écoulement (8.1), dans le flux d'air principal (HL) du système de climatisation (1), et le flux d'air partiel (TLO) chargé en gaz est mélangé avec le flux d'air principal (HL), dans le conduit de guidage d'air (1.4), de manière telle qu'un composant (1.1) du système de climatisation (1) soit alimenté, de façon homogène, avec le flux d'air principal (HLO) chargé en gaz, **caractérisé en ce que** le flux d'air partiel (TLO) chargé en gaz est fourni au flux d'air principal (HL), par plusieurs entrées d'écoulement (8.1).

9. Procédé selon la revendication 8, où le flux d'air partiel (TLO) chargé en gaz est introduit dans le flux d'air principal (HL), en amont d'un composant du système de climatisation (1), configuré comme un évaporateur (1.1).

10. Procédé selon l'une des revendications 8 et 9, où le flux d'air partiel (TLO) chargé en gaz est réparti sur une conduite annulaire (8), au moyen d'un ventilateur auxiliaire (10), et introduit dans le conduit de guidage d'air (1.4), par plusieurs buses (8.1) disposées dans la conduite annulaire (8).

11. Procédé selon l'une quelconque des revendications 8 à 10, où le flux d'air partiel (TL) est dévié dans la conduite de dérivation (4), en amont d'un composant (1.6) du système de climatisation (1), déclenchant une chute de pression.

12. Procédé selon l'une quelconque des revendications 8 à 11, où le flux d'air partiel (TL) est dévié dans la conduite de dérivation (4) à partir d'une zone du conduit de guidage d'air (1.4) qui présente une chute de pression dynamique.
